# EUROPÄISCHE PATENTSCHRIFT

(11) **EP 0 695 726 B1**
(45) Veröffentlichungstag und Bekanntmachung des Hinweises auf die Patenterteilung: **02.07.2003**
(21) Anmeldenummer: 95250187.2
(22) Anmeldetag: 31.07.1995
(51) Int. Cl.: C03C 10/00, C03C 3/085, A61K 6/06

(54) **Alkali-Zink-Silicat-Glaskeramiken und -Gläser**
Alkali zinc silicate glass ceramics and glasses
Vitrocéramiques et verre de silicate d'alkali et de zinc

(30) Priorität: 01.08.1994 DE 4428839
(43) Veröffentlichungstag der Anmeldung: 07.02.1996
(60) Teilanmeldung: 03008836.3
(73) Patentinhaber: Ivoclar Vivadent AG, 9494 Schaan (LI)
(72) Erfinder: Frank, Martin, FL-9494 Schaan (LI); Wegner, Susanne, D-88131 Lindau (DE); Rheinberger, Volker, FL-9490 Vaduz (LI); Höland, Wolfram, FL-9494 Schaan (LI)
(74) Vertreter: UEXKÜLL & STOLBERG

(56) Entgegenhaltungen:
- EP-A- 0 622 342
- DE-A- 2 034 393
- GB-A- 1 022 681
- US-A- 3 907 577
- DATABASE WPI Section Ch, Week 8112 Derwent Publications Ltd., London, GB; Class D21, AN 81-20178D & JP-A-56 007 708 ( HOYA GLASS WORKS) , 27.Januar 1981

## Beschreibung

Die Erfindung betrifft Alkali-Zink-Silicat-Glaskeramiken und insbesondere solche, die sich aufgrund ihrer vorteilhaften Eigenschaften, wie im Bereich von 8,0 bis 18,7 x 10⁻⁶ K⁻¹ einstellbaren linearen thermischen Ausdehnungskoeffizienten und niedrigen Verarbeitungstemperaturen als Dentalmaterial eignen.

In der Zahnheilkunde werden metallische Dentalrestaurationen üblicherweise mit keramischen Schichten verblendet, um das Aussehen der Restauration an das der natürlichen Zähne anzugleichen. Solche verblendeten Restaurationen werden auch als Verblendkeramiken oder Metallkeramiken bezeichnet. Um nun Spannungen zwischen dem Metallgerüst und der Keramikschicht zu vermeiden, ist es erforderlich, daß die Wärmeausdehnungskoeffizienten der keramischen Werkstoffe an die des Metalls angepaßt sind.

Es ist bekannt, daß leucithaltige Glaskeramiken sehr hohe lineare thermische Ausdehnungskoeffizienten besitzen. Diese sind auf den Gehalt an Leucit zurückzuführen, welches durch gesteuerte Kristallisation aus einem entsprechenden Ausgangsglas gebildet wird.

Damit eine Dentalglaskeramik zum Verblenden der gesamten Bandbreite von verwendeten Dentalmetallen und -legierungen, wie z.B. Titan bis hin zu Legierungen mit hohem Goldgehalt, eingesetzt werden kann, ist es erforderlich, daß ihr Ausdehnungskoeffizient in einem breiten Bereich einstellbar ist. Sofern die Dentalglaskeramik zudem auch noch als Korrekturmaterial für Aufbrennkeramiken Verwendung finden soll, so sind weiter insbesondere tiefe Sintertemperaturen von weniger als 880°C und geeignete optische Eigenschaften, wie hohe Transluzenz, sehr wünschenswert.

Bekannte Glaskeramiken und Gläser erfüllen die Forderung nach in einem weiten Bereich einstellbaren thermischen Ausdehnungskoeffizienten und einer niedrigen Verarbeitungstemperatur häufig nicht. Zudem weisen die bekannten Dentalwerkstoffe in vielen Fällen physiologisch nicht völlig unbedenkliche Komponenten, wie z.B. Sb₂O₃, auf oder es ist zwingend erforderlich, ihnen B₂O₃ zuzusetzen, um die für Dentalwerkstoffe gewünschten Eigenschaften zu erzielen. Nach Untersuchungen der Erfinder an Grundgläsern des Systems SiO₂-Al₂O₃-Na₂O-K₂O führen geringe B₂O₃-Zusätze von ca. 3 Gew.-% zu einer unannehmbaren Verschlechterung der chemischen Beständigkeit und hohe B₂O₃-Gehalte von ca. 12 Gew.-% zu einem zu geringen Ausdehnungskoeffizienten.

Dentalkeramische Werkstoffe mit Gehalt an B₂O₃ sind z.B. aus der DE-OS 39 11 460 und der DE-PS 41 38 875 bekannt. Diese Werkstoffe besitzen relativ niedrige Verarbeitungstemperaturen und können zur Verblendung von Dentallegierungen eingesetzt werden. Ihr Wärmeausdehnungskoeffizient kann jedoch lediglich im Bereich von ungefähr 13 bis 14 x 10⁻⁶ K⁻¹ eingestellt werden.

Weiter enthalten die Werkstoffe zwingend Sb₂O₃, hingegen kein ZnO und kein ZrO₂.

Ein keramischer Werkstoff zum Verblenden von metallischem Zahnersatz ist ebenfalls aus der DE-OS 40 31 168 bekannt. Auch wenn der Ausdehnungskoeffizient dieses Werkstoffes in einem Bereich von 8 bis 17,5 × 10⁻⁶ K⁻¹ einstellbar sein soll, so enthält der Werkstoff dennoch 0,7 bis 2,5 Gew.-% B₂O₃. Weiter ist in dem Werkstoff kein ZnO und kein ZrO₂ vorhanden.

Porzellanmassen mit einem hohen Gehalt an B₂O₃ von 7 bis 33 Gew.% und Verarbeitungstemperaturen im Bereich von 800°C sind aus der US-PS 5 176 747 bekannt. Diese Massen können als Dentalporzellan zur Verblendung von Titan oder Titan-Legierungen eingesetzt werden. Das verwendete B₂O₃ dient dabei sowohl zur Herabsetzung der Verarbeitungstemperatur als auch zur Herabsetzung des thermischen Ausdehnungskoeffizienten. Darüber hinaus wird dem B₂O₃ auch ein Einfluß auf die Bindungsstärke zwischen Metallsubstrat und Keramik beigemessen. Ähnliche keramische Materialien mit einem Gehalt von 8 bis 17 Gew.-% B₂O₃ werden in der EP-A-468 435 beschrieben. Diese Materialien enthalten kein ZnO und können ebenfalls zur Verblendung von Dentalrestaurationen, wie Kronen, Brücken oder Prothesenteilen eingesetzt werden, welche aus Titan oder Titan-Legierungen hergestellt sind.

Weiter sind aus der EP-A-0 155 564 leucithaltige Glaskeramiken bekannt, die jedoch B₂O₃ und physiologisch nicht unbedenkliches Sb₂O₃ enthalten.

Der Erfindung liegt nunmehr die Aufgabe zugrunde, Glaskeramiken zu schaffen, die bei niedrigen Temperaturen durch Sinterung verarbeitbar sind, einen im Bereich von insbesondere 8,0 bis 18,7 × 10⁻⁶ K⁻¹ einstellbaren linearen thermischen Ausdehnungskoeffizienten besitzen und gleichzeitig vorteilhafte optische Eigenschaften, wie hohe Transluzenz und Opalenszenz, sowie eine ausgezeichnete chemische Beständigkeit haben und dabei ohne Zusatz von B₂O₃ und/oder physiologisch nicht völlig unbedenkliche Komponenten erzeugt werden können und demgemäß sich in vorteilhafter Weise zur Anwendung in der Dentaltechnik eignen.

Diese Aufgabe wird durch die Alkali-Zink-Silicat-Glaskeramik nach den Ansprüchen 1 bis 7 gelöst.

Gegenstand der vorliegenden Erfindung sind ebenfalls ein Verfahren zur Herstellung der Glaskeramik, die Verwendung der Glaskeramik ein Dentalmaterial sowie geformte Dentalprodukte mit Gehalt an der Glaskeramik .

Die erfindungsgemäße Alkali-Zink-Silicat-Glaskeramik ist dadurch gekennzeichnet, daß sie die folgenden Komponenten enthält

| Komponente | Gew.- % |
|---|---|
| SiO₂ | 52,0 bis 63,5 |
| Me(III)₂O₃ | 8,5 bis 13,0 |
| K₂O | 0 bis 20,5 |
| Na₂O | 1,5 bis 20,0 |
| Li₂O | 0 bis 5,0 |
| ZnO 2,0 bis 8,0, insbesondere | 3,1 bis 8,0 |
| Me(II)O | 2,5 bis 6,5 |
| TiO₂ + ZrO₂ | 0,5 bis 6,0 |
| SnO₂ | 0 bis 9,5 |
| P₂O₅ | 0 bis 4,0 |
| F | 0 bis 2,0 |
| CeO₂ | 0 bis 3,0 |

wobei
a) die angegebene Menge Me(III)₂O₃ aus 0 bis 13 Gew.-% Al₂O₃ und 0 bis 9,5 Gew.-% La₂O₃; und
b) die angegebene Menge Me(II)O aus 0 bis 3,5 Gew.-% CaO, 0 bis 4,5 Gew.-% BaO und 0 bis 5,0 Gew.-% MgO
gebildet ist.

Vorzugsweise besteht die Glaskeramik im wesentlichen aus den zuvor genannten Komponenten.

Für einige der Komponenten der Glaskeramik existieren bevorzugte Mengenbereiche. Diese können unabhängig voneinander gewählt werden und sind wie folgt:

| Komponente | Gew.-% |
|---|---|
| SiO₂ | 52,0 bis 61,0 |
| Al₂O₃ | 8,5 bis 11,0 |
| La₂O₃ | 0 bis 2,0 |
| K₂O | 0 bis 15,0 |
| Na₂O | 6,0 bis 15,0 |
| Li₂O | 0 bis 4,0 |
| ZnO 3,6 bis 7,0, insbesondere | 4,0 bis 7,0 |
| CaO | 0,5 bis 3,5 |
| BaO | 1,0 bis 4,5 |
| TiO₂ | 0 bis 2,8 |
| ZrO₂ | 0,5 bis 5,0 |
| P₂O₅ | 0 bis 2,0 |

Es ist ganz besonders bevorzugt, daß die erfindungsgemäße Glaskeramik im wesentlichen frei von B₂O₃, Antimon- und/oder Bleiverbindungen ist.

Zur Herstellung eines Glases, welches die Komponenten der erfindungsgemäßen Glaskeramik enthält, wird vorzugsweise so vorgegangen, daß geeignete Ausgangsmaterialien, wie z.B. Carbonate, Oxide und Fluoride, bei einer Temperatur im Bereich von 1350 bis 1650°C, vorzugsweise 1400 bis 1600°C, über einen Zeitraum von 30 Minuten bis 4 Stunden, vorzugsweise eine Stunde bis 2,5 Stunden, unter Bildung einer homogenen Schmelze erschmolzen werden. Das erschmolzene Glas wird anschließend in Wasser abgeschreckt, d.h. gefrittet, und das erhaltene Glasgranulat wird nach Trocknen aufgemahlen.

Zur Herstellung der erfindungsgemäßen Glaskeramik wird insbesondere so vorgegangen, daß das erhaltene Granulat des Glases einer thermischen Behandlung bei einer Temperatur im Bereich von 600 bis 900°C für eine Dauer von 30 Minuten bis 5 Stunden, vorzugsweise 30 Minuten bis 2 Stunden, unterzogen wird. Vorzugsweise wird das hierbei eingesetzte Glas vor der Wärmebehandlung zu einem Pulver mit einer Korngröße von weniger als 90 µm gemahlen und gesiebt. Die thermische Behandlung kann auch durch die im Rahmen der Herstellung von geformten Dentalprodukten aus dem Glas erforderlichen Wärmebehandlungen, wie Sinterungsvorgänge, bewirkt werden.

Mittels rasterelektronenmikroskopischer Untersuchungen wurde festgestellt, daß die Gläser frei von Kristallen sind oder nur sehr vereinzelt Kristalle aufweisen. Hingegen enthalten die erfindungsgemäßen Glaskeramiken Kristalle, insbesondere Leucitkristalle, welche durch die gesteuerte Oberflächenkristallisation im Rahmen der thermischen Behandlung gebildet wurden. Vorzugsweise bilden die Leucitkristalle die Hauptkristallphase in den Glaskeramiken, und die mittlere Größe der Leucitkristalle beträgt vorzugsweise weniger als 5 µm bezogen auf die Anzahl der Kristalle.

Zusätzlich zu Leucitkristallen können je nach chemischer Zusammensetzung des eingesetzten Glases weitere Kristallphasen gebildet werden. Neben den verschiedenen Kristallphasen können auch mikroheterogene Entmischungsbereiche, d.h. verschiedene Glasphasen, vorliegen. Diese Bereiche sind im Rasterelektronenmikroskop als kleine mikroheterogene Tropfenglasphasen mit einer Größe von ca. 40 bis 250 nm erkennbar. Das Vorliegen dieser Tropfenglasphasen oder von Kristallen beeinflußt die optischen Eigenschaften, wie z.B. Opaleszenz und Transluzenz, der erfindungsgemäßen Glaskeramiken.

Der lineare thermische Ausdehnungskoeffizient der erfindungsgemäßen Glaskeramiken kann vorzugsweise im Bereich von 8,0 bis 18,7 × 10⁻⁶ K⁻¹, gemessen im Temperaturbereich von 100 bis 400°C, eingestellt werden. Es ist überraschend, daß trotz des hohen Gehalts von 52,5 bis 63,5 Gew.-% SiO₂ und ohne Zusatz von B₂O₃ den erfindungsgemäßen Glaskeramiken sowohl hohe als auch niedrige Ausdehnungskoeffizienten verliehen werden können. Nach dem Stand der Technik ist zur Einstellung von niedrigen Ausdehnungskoeffizienten hingegen ein Zusatz von B₂O₃ in den meisten Fällen zwingend erforderlich.

Durch die Verwendung von einwertigen Netzwerkwandlerionen, wie Kalium, Natrium und Lithium, und durch Einsatz von Fluor konnte die Verarbeitungstemperatur gesenkt werden. So können die erfindungsgemäßen Glaskeramiken in Pulverform bei Temperaturen von vorzugsweise 640 bis 850°C zusammengesintert und damit verarbeitet werden.

Der hohe Gehalt an ZnO in den erfindungsgemäßen Glaskeramiken trägt wesentlich zur guten chemischen Beständigkeit bei und reduziert im Vergleich zu ZrO₂ deren Viskosität. Darüber hinaus zeichnet sich ZnO durch eine ausgezeichnete physiologische Verträglichkeit aus.

Neben den zuvor erwähnten Komponenten können die erfindungsgemäßen Glaskeramiken außerdem Zusatzstoffe, wie z.B. Farbstoffe, insbesondere Farbpigmente, Oxide der 3d-Elemente oder Metallkolloide, oder Fluoreszenzstoffe, insbesondere mit d- und f-Elementen dotiertes Ytterbium-Silicat, enthalten. Weitere geeignete Zusatzstoffe zur Veränderung von z.B. den optischen und/oder thermischen Eigenschaften der erfindungsgemäßen Glaskeramiken sind weitere Gläser, Keramiken, weitere Glaskeramiken, Trübungsstoffe und/oder Stabilisatoren.

Die erfindungsgemäßen Glaskeramiken können entweder allein als Dentalmaterial oder als Bestandteil von Dentalmaterialien, wie z.B. Dentalkeramikpulvern, eingesetzt werden. Hierzu wird die erfindungsgemäße Glaskeramik vorzugsweise in Form eines Pulvers mit einer Teilchengröße von insbesondere weniger als 90 µm eingesetzt. Dieses Glaskeramik-Pülver eignet sich dabei in besonders vorteilhafter Weise als Korrekturmaterial für metallkeramische oder vollkeramische Dentalsuprastrukturen, wie z.B. eine Teilkrone, eine Krone oder eine Brücke. Für diesen Zweck wird das Pulver auf die gewünschten Stellen der Dentalsuprastruktur aufgebracht und bei Temperaturen von 640 bis 850°C in einem Vakuumbrennofen zusammengesintert. Dabei können die Eigenschaften des Pulvers, wie z.B. thermischer Ausdehnungskoeffizient und optische Eigenschaften an die des jeweils aufgesinterten Basismaterials angepaßt werden. Ein bevorzügtes Dentalmaterial enthält Alkalizink - silicat- Glas und erfindungsgemäße Glaskeramik, wobei das Glas die Komponenten der erfindüngsgemäßen Glaskeramic enthält.

In einer weiteren, bevorzugten Ausführungsform der Erfindung kann die erfindungsgemäße Glaskeramik auch für sich als Schicht- oder Verblendmaterial für vollkeramische, metallische oder in Form von Legierungen vorliegende Dentalsuprastrukturen verwendet werden. Hierzu wird die gepulverte Glaskeramik mit Wasser vermischt und auf das metallische oder vollkeramische Substrat aufgebracht. Nach Formung der gewünschten Dentalrestauration, wie z.B. Brücke oder Krone, wird diese bei Temperaturen von 640 bis 850°C zu dem fertigen, geformten Dentalprodukt dichtgesintert. Dabei ist es von besonderem Vorteil, daß der thermische Ausdehnungskoeffizient der

Glaskeramik innerhalb eines breiten Bereichs variiert werden kann, und zwar auf 8,0 × 10⁻⁶ K⁻¹ für ein Titansubstrat und ungefähr 16,0 × 10⁻⁶ K⁻¹ für ein Substrat aus Gold oder Legierung mit hohem Goldgehalt eingestellt und damit an den Ausdehnungskoeffizienten des verwendeten Substrats angepaßt werden kann.

Es ist besonders überraschend, daß die erfindungsgemäßen Glaskeramiken eine Kombination von niedriger Verarbeitungstemperatur, in einem breiten Bereich einstellbaren thermischen Ausdehnungskoeffizienten und sehr gute chemische Beständigkeit aufweisen.

Als erfindungsgemäße geformte Dentalprodukte, die einen Gehalt an der erfindungsgemäßen Glaskeramik aufweisen, kommen insbesondere Dentalrestaurationen, wie z.B. Kronen, Teilkronen und Brücken, in Frage.

Die Erfindung wird nachstehend anhand von Beispielen näher erläutert.

### Beispiele

### Beispiele 1 bis 21

Es wurden insgesamt 21 verschiedene erfindungsgemäße Glaskeramiken und 21 verschiedene Gläser hergestellt. Sie hatten die in der Tabelle I angegebenen chemischen Zusammensetzungen.

Für einige der Glaskeramiken und Gläser sind in Tabelle II ausgewählte Eigenschaften angegeben, die an Probekörpern aus dem jeweiligen Glas oder der jeweiligen Glaskeramik bestimmt worden sind. Weiter finden sich in Tabelle II unter "Temperaturbehandlung" Angaben zu dem jeweils eingesetzten Ausgangsmaterial für die Probekörper sowie Angaben zu einer etwaigen Wärmebehandlung des Ausgangsmaterials. Bei allen Ausgangsmaterialien, für die keine Wärmebehandlung angegeben ist, handelte es sich um Gläser. Die Ausgangsmaterialien mit angegebener Wärmebehandlung waren erfindungsgemäße Glaskeramiken. Es ist jedoch zu beachten, daß im Falle des nicht-wärmebehandelten Glases Nr. 12 eine Umwandlung zu einer entsprechenden Glaskeramik eintrat, wenn dieses im in Tabelle II angegebenen Temperaturbereich zu Probekörpern gesintert wurde.

Die Tabelle II zeigt weiter, daß eine Glaskeramik in der Regel einen höheren Ausdehnungskoeffizienten als ein Glas entsprechender chemischer Zusammensetzung hat.

Die Beispiele verdeutlichen wie durch Veränderung der chemischen Zusammensetzung und durch etwaige Wärmebehandlung Glaskeramiken und Gläser mit unterschiedlichen Eigenschaften erhalten werden können.

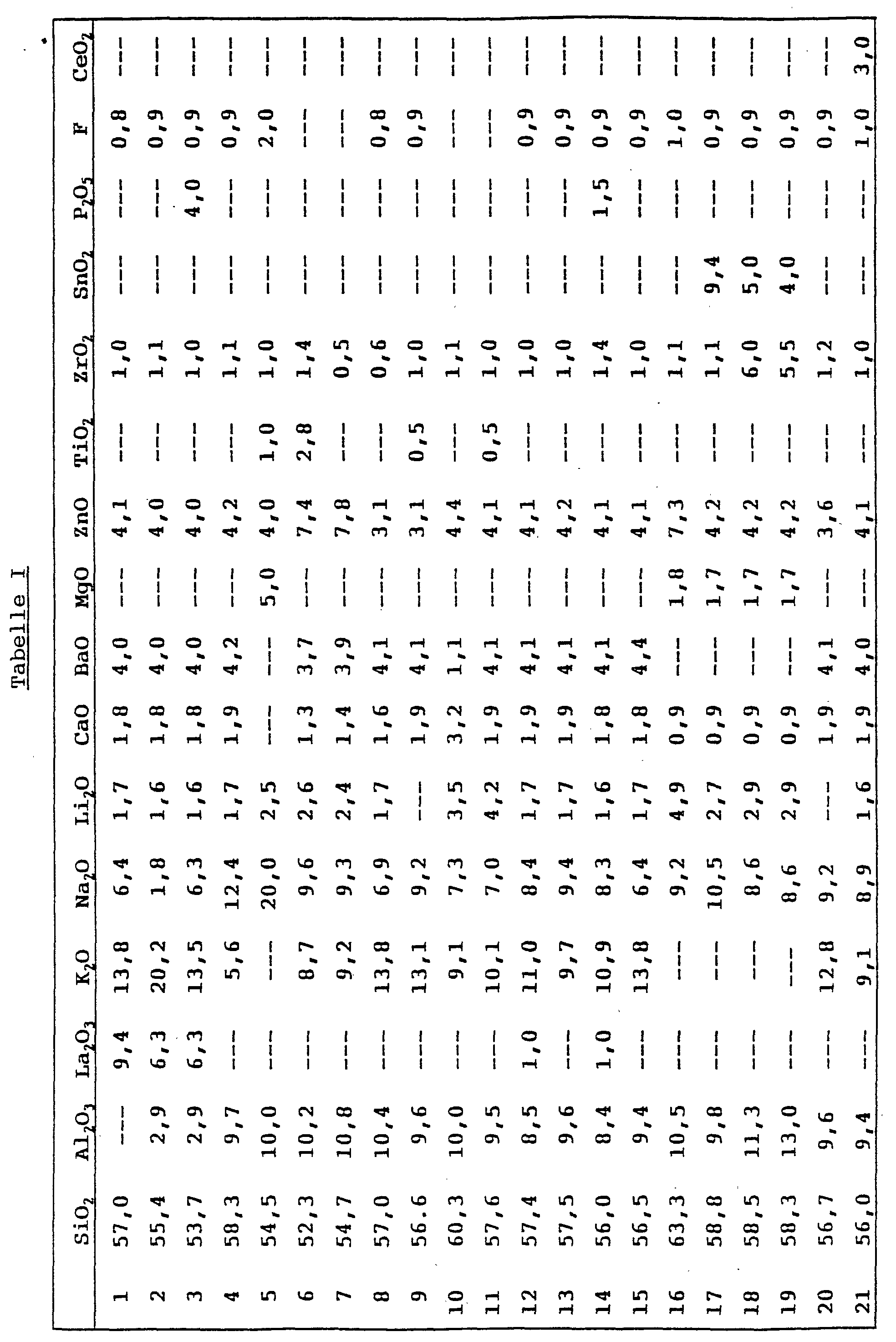

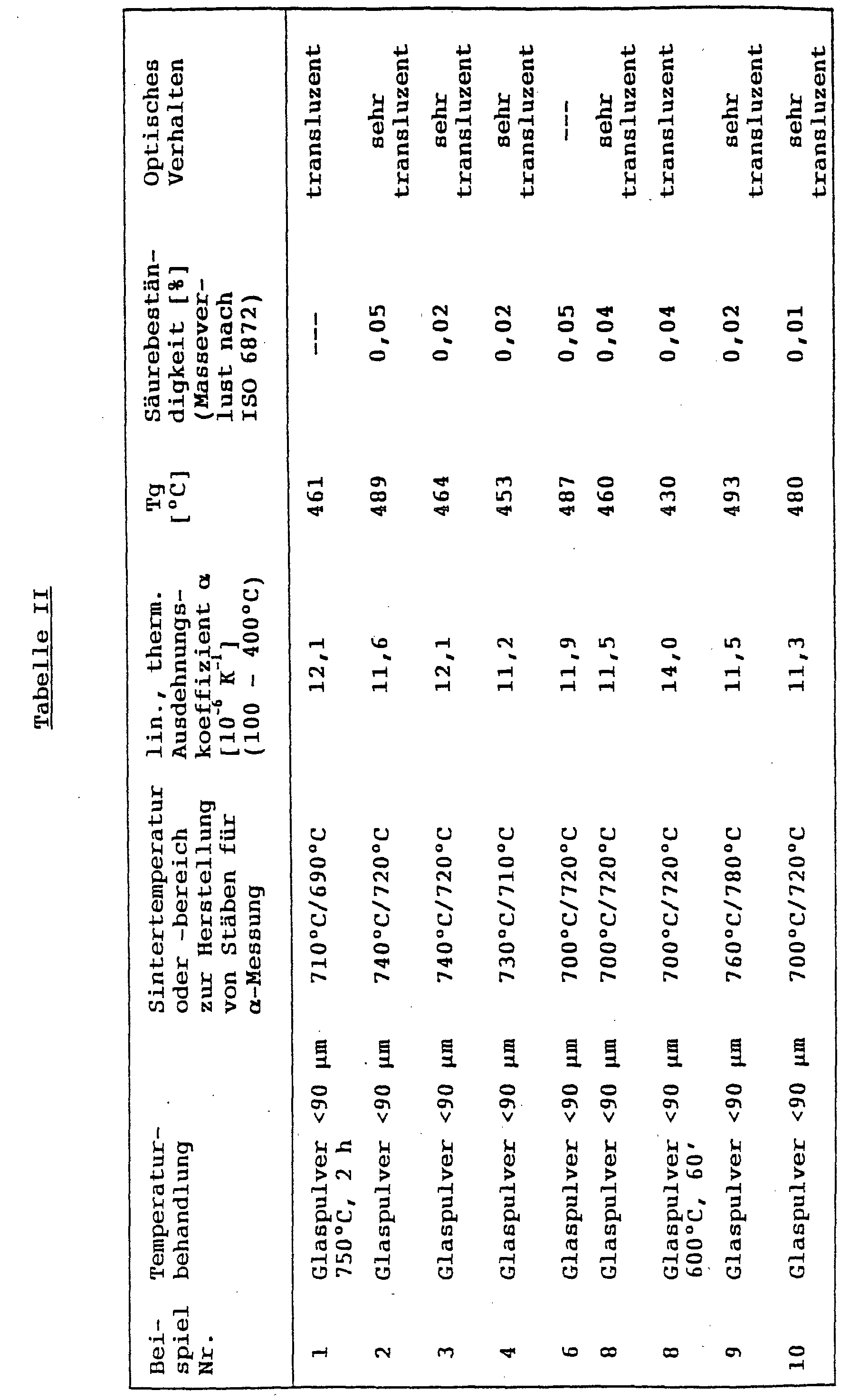

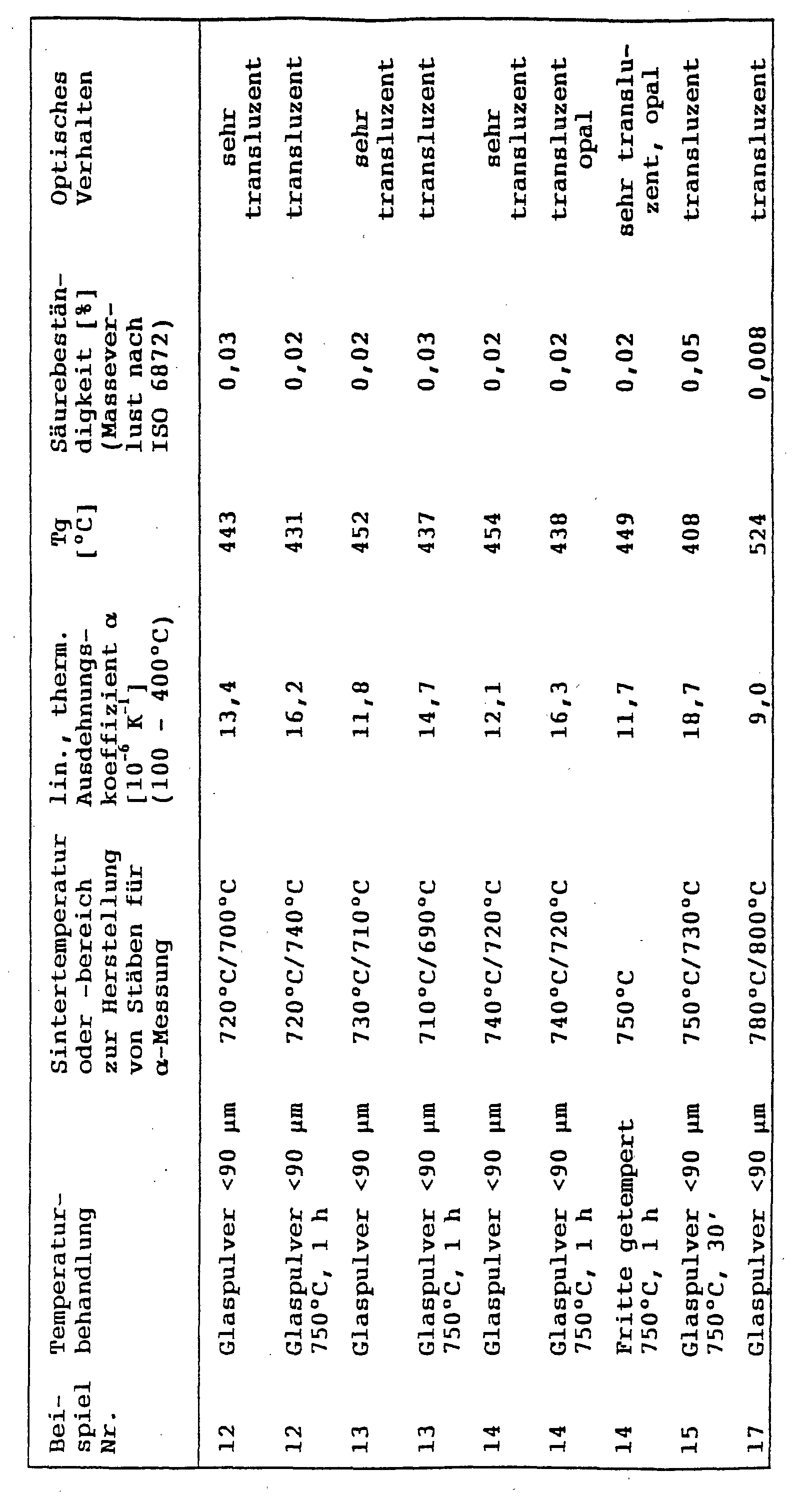

### Beispiel 22

Dieses Beispiel beschreibt die Herstellung einer erfindungsgemäßen Glaskeramik, die als tiefschmelzende Verblendkeramik oder als Korrekturmaterial von sowohl Verblend- als auch Vollkeramiken verwendet werden kann.

Zunächst wurde ein Ausgangsglas mit der in Tabelle I für Beispiel 12 angegebenen chemischen Zusammensetzung hergestellt. Dazu wurde ein Gemenge von Oxiden, Carbonaten und Fluoriden in einem Platin/Rhodium-Tiegel bei einer Temperatur von 1550 bis 1600°C während einer Homogenisierungszeit von ungefähr 2 Stunden erschmolzen. Die Glasschmelze wurde in Wasser abgeschreckt, und die gebildete Glasfritte wurde getrocknet und auf eine Korngröße von weniger als 90 µm aufgemahlen. Anschließend wurde das erhaltene Glaspulver eine Stunde lang bei 750°C wärmebehandelt, erneut aufgemahlen und auf eine Teilchengröße von weniger als 90 µm gesiebt. Mit dem erhaltenen Glaskeramikpulver wurden Prüfkörper hergestellt und die in Tabelle II unter Nr. 12, mit Wärmebehandlung, angegebenen Eigenschaften bestimmt.

Zur Messung des linearen thermischen Ausdehnungskoeffizienten wurde aus dem Glaskeramikpulver ein stäbchenförmiger Grünkörper hergestellt, der in einem Vakuumbrennofen mit einer Aufheizrate von 60°C/min und einer Haltezeit von 1 Minute bei einer Temperatur von 720°C gebrannt wurde. Anschließend wurde ein Glanzbrand ohne Vakuum bei einer Endtemperatur von 740°C und einer Haltezeit von 1 Minute durchgeführt. Der lineare thermische Ausdehnungskoeffizient für den erhaltenen Probekörper betrug 16,2 × 10⁻⁶ K⁻¹, gemessen im Temperaturbereich von 100 bis 400°C. Damit ist der Ausdehnungskoeffizient dieser Glaskeramik an den von Legierungen mit hohem Goldgehalt angepaßt.

Auch bei der Herstellung von Plättchen anstelle von Stäben konnte die Glaskeramik bei einer sehr niedrigen Temperatur von nur 740°C zusammengesintert werden. Die Herstellung der Plättchen erfolgte in der Weise, daß aus dem Glaskeramikpulver ein Grünling in Plättchenform gebildet und dieser auf einem mit Quarzmehl bestrichenen Brenngutträger im Vakuumofen bei 740°C und einer Haltezeit von einer Minute gebrannt wurde. Die Aufheizrate des Vakuumofens betrug dabei 60°C/min.

Die Bestimmung der chemischen Beständigkeit der Glaskeramik durch Behandlung von gesinterten Plättchen mit 4%iger Essigsäure in einer Soxhlet-Apparatur gemäß ISO 6872 führte zu einem sehr geringen Masseverlust der Glaskeramik von nur 0,02%.

Durch den an Legierungen mit hohem Goldgehalt angepaßten linearen thermischen Ausdehnungskoeffizienten, die sehr gute chemische Beständigkeit und die niedrige Verarbeitungstemperatur ist diese Glaskeramik besonders zum Aufsintern auf solche Legierungen sowie als Korrekturmaterial für Verblend- und Vollkeramiken geeignet.

Zur Erzielung von linearen thermischen Ausdehnungskoeffizienten, die auf alle derzeit üblichen goldhaltigen Dentallegierungen angepaßt sind, kann die Glaskeramik vorzugsweise mit anderen gepulverten erfindungsgemäßen Glaskeramiken und Gläsern einer in Tabelle I angegebenen chemischen Zusammensetzung gemischt werden.

### Beispiel 23

Dieses Beispiel beschreibt die Herstellung einer erfindungsgemäßen Glaskeramik, die als Korrekturmaterial für Vollkeramiken und insbesondere für Metallkeramiken eingesetzt werden kann.

Zunächst wurde ein Glas mit der in Tabelle I für Beispiel 13 angegebenen Zusammensetzung entsprechend der in Beispiel 22 beschriebenen Verfahrensweise erschmolzen und aufgemahlen. Das erhaltene Pulver wurde als Pulver I bezeichnet.

Weiter wurde ein Glas mit der in Tabelle I für Beispiel 14 angegebenen Zusammensetzung ebenfalls entsprechend der Verfahrensweise gemäß Beispiel 22 erschmolzen und gefrittet. Die getrocknete Fritte wurde dann eine Stunde lang bei 750°C wärmebehandelt und schließlich in einer Achatmühle aufgemahlen und auf eine Teilchengröße von weniger als 90 µm gesiebt. Das erhaltene Pulver wurde als Pulver II bezeichnet.

Durch geeignetes Mischen dieser zwei Pulver mit dem Glaskeramikpulver gemäß Beispiel 22 konnte der Ausdehnungskoeffizient in gewünschter Weise eingestellt und damit die erhaltene Mischung als Korrekturmaterial zum Aufsintern auf Metallkeramikkronen mit sehr guten optischen Eigenschaften verwendet werden. Beispielsweise bestand eine geeignete Mischung aus 70 Gew.-% Pulver I, 15 Gew.-% Pulver II und 15 Gew.-% Pulver gemäß Beispiel 22, und diese Mischung hatte einen Ausdehnungskoeffizienten von 12,7 x 10⁻⁶ K⁻¹.

Zur Verwendung als Korrekturmaterial wurde diese Mischung auf die zu korrigierende Stelle einer Metallkeramikkrone aufgebracht, und die Krone wurde bei einer Temperatur von 640°C gebrannt, wobei ab 580°C unter Vakuum gearbeitet wurde, die Aufheizrate 60°C/min und die Haltezeit eine Minute betrug. Die fertige Krone hatte an der korrigierten Stelle ein sehr transluzentes und insbesondere im Schneidebereich leicht opaleszentes Aussehen und wirkte dadurch lebhaft.

Bei einer Temperatur von 730°C und einer Haltezeit von einer Minute konnten aus dieser Mischung Plättchen auf Quarzmehl gebrannt werden. Außerdem zeigten gemäß ISO 6872 hergestellte und geprüfte Plättchen aus dieser Mischung eine sehr gute Säurebeständigkeit von lediglich 0,02% Masseverlust.

### Beispiel 24

Dieses Beispiel beschreibt eine erfindungsgemäße Glaskeramik, die als Korrekturmaterial für Verblendkeramiken und insbesondere für Vollkeramikkronen verwendet werden kann.

Zunächst wurde ein Glas mit der in Tabelle I für Beispiel 15 angegebenen chemischen Zusammensetzung gemäß der in Beispiel 22 beschriebenen Verfahrensweise erschmolzen und gemahlen. Das erhaltene Glaspulver wurde dann 30 Minuten lang bei 750°C wärmebehandelt. Die Eigenschaften der erhaltenen Glaskeramik sind in Tabelle II unter Nr. 15 angegeben.

Durch geeignetes Mischen eines Pulvers dieser Glaskeramik mit dem Pulver I gemäß Beispiel 23 konnte der Ausdehnungskoeffizient so eingestellt werden, daß die erhaltene Mischung als Korrekturmaterial zum Aufsintern auf vollkeramische Kronen verwendet werden konnte. Eine für diesen Zweck geeignete Mischung enthielt 80 Gew.-% des Glaskeramikpulvers und 20 Gew.-% Glaspulver I gemäß Beispiel 23 und hatte einen Ausdehnungskoeffizienten von 16,7 × 10⁻⁶ K⁻¹, gemessen im Bereich von 100 bis 400°C.

## Patentansprüche

1. Alkali-Zink-Silicat-Glaskeramik, **dadurch gekennzeichnet, daß** sie die folgenden Komponenten enthält:
| Komponente | Gew.-% |
|---|---|
| SiO₂ | 52,0 bis 63,5 |
| Me(III)₂O₃ | 8,5 bis 13,0 |
| K₂O | 0 bis 20,5 |
| Na₂O | 1,5 bis 20,0 |
| Li₂O | 0 bis 5,0 |
| ZnO 2,0 bis 8,0, insbes. | 3,1 bis 8,0 |
| Me(II)O | 2,5 bis 6,5 |
| TiO₂ + ZrO₂ | 0,5 bis 6,0 |
| SnO₂ | 0 bis 9,5 |
| P₂O₅ | 0 bis 4,0 |
| F | 0 bis 2,0 |
| CeO₂ | 0 bis 3,0 |
wobei
a) die angegebene Menge Me(III)₂O₃ aus 0 bis 13 Gew.-% Al₂O₃ und 0 bis 9,5 Gew.-% La₂O₃; und
b) die angegebene Menge Me(II)O aus 0 bis 3,5 Gew.-% CaO, 0 bis 4,5 Gew.-% BaO und 0 bis 5,0 Gew.-% MgO
gebildet ist.

2. Glaskeramik nach Anspruch 1, **dadurch gekennzeichnet, daß** die Mengen von einigen Komponenten unabhängig von einander wie folgt sind:
| Komponente | Gew.-% |
|---|---|
| SiO₂ | 52,0 bis 61,0 |
| Al₂O₃ | 8, 5 bis 11,0 |
| La₂O₃ | 0 bis 2,0 |
| K₂O | 0 bis 15,0 |
| Na₂O | 6,0 bis 15,0 |
| Li₂O | 0 bis 4,0 |
| ZnO 3,6 bis 7,0, insbesondere | 4,0 bis 7,0 |
| CaO | 0,5 bis 3,5 |
| BaO | 1,0 bis 4,5 |
| TiO₂ | 0 bis 2,8 |
| ZrO₂ | 0,5 bis 5,0 |
| P₂O₅ | 0 bis 2,0 |

3. Glaskeramik nach Anspruch 1 oder 2, **dadurch gekennzeichnet, daß** sie im wesentlichen frei von B₂O₃ ist.

4. Glaskeramik nach einem der Ansprüche 1 bis 3, **dadurch gekennzeichnet, daß** sie Leucitkristalle aufweist.

5. Glaskeramik nach Anspruch 4, **dadurch gekennzeichnet, daß** die Leucitkristalle eine mittlere Größe von weniger als 5 µm haben, bezogen auf die Anzahl der Kristalle.

6. Glaskeramik nach einem der Ansprüche 1 bis 5, **dadurch gekennzeichnet, daß** sie als Zusatzstoffe Farbstoffe, Fluoreszenzstoffe, weitere Gläser, Keramiken, weitere Glaskeramiken, Trübungsstoffe und/oder Stabilisatoren enthält.

7. Glaskeramik nach einem der Ansprüche 1 bis 6, **dadurch gekennzeichnet, daß** sie einen linearen thermischen Ausdehnungskoeffizienten von 8,0 bis 18,7 x 10⁻⁶ K⁻¹, gemessen im Bereich von 100 bis 400°C, hat.

8. Verfahren zur Herstellung der Glaskeramik nach einem der Ansprüche 1 bis 7, **dadurch gekennzeichnet, daß** ein Glas hergestellt wird, welches die Komponenten gemäß Anspruch 1 enthält, und das hergestellte Glas anschließend einer Wärmebehandlung bei 600 bis 900°C über einen Zeitraum von 30 Minuten bis 5 Stunden unterworfen wird.

9. Verfahren nach Anspruch 8, **dadurch gekennzeichnet, daß** das hergestellte Glas vor der Wärmebehandlung zu einem Pulver mit einer Korngröße von weniger als 90 µm gemahlen und gesiebt wird.

10. Dentalmaterial das Alkali-Zink-Silicat-Glas und Glaskeramik gemäß Ansprüch 1 enthält, wobei das Alkali-Zink-Silicat-Glas die Komponenten gemäß Anspruch 1 enthält.

11. Verwendung der Glaskeramik gemäß einem der Ansprüche 1 bis 7 als Dentalmaterial oder Bestandteil von Dentalmaterial.

12. Verwendung nach Anspruch 11, wobei die Glaskeramik oder das Glas als Korrekturmaterial für metallkeramische oder vollkeramische Dentalsuprastrukturen eingesetzt wird.

13. Verwendung nach Anspruch 11 oder 12, wobei die Dentalsuprastruktur die Form einer Krone, einer Brücke oder einer Teilkrone hat.

14. Verwendung nach einem der Ansprüche 11 bis 13, wobei die Glaskeramik als Korrekturmaterial für Inlays eingesetzt wird.

15. Verwendung nach einem der Ansprüche 11 bis 13, wobei die Glaskeramik als Verblendmaterial für vollkeramische, metallische oder in Form von Legierungen vorliegende Dentalsuprastrukturen eingesetzt wird.

16. Geformtes Dentalprodukt, welches einen Gehalt an der Glaskeramik gemäß einem der Ansprüche 1 bis 7 aufweist.

## Claims

1. Alkali-zinc-silicate glass ceramic, **characterised in that** it comprises the following components:
| Component | wt. % |
|---|---|
| SiO₂ | 52.0 to 63.5 |
| Me(III)₂O₃ | 8.5 to 13.0 |
| K₂O | 0 to 20.5 |
| Na₂O | 1.5 to 20.0 |
| Li₂O | 0 to 5.0 |
| ZnO 2.0 to 8.0, in particular | 3.1 to 8.0 |
| Me(II)O | 2.5 to 6.5 |
| TiO₂ + ZrO₂ | 0.5 to 6.0 |
| SnO₂ | 0 to 9.5 |
| P₂O₅ | 0 to 4.0 |
| F | 0 to 2.0 |
| CeO₂ | 0 to 3.0 |
where
a) the quantity of Me(III)₂O₃ given is formed from 0 to 13 wt.% Al₂O₃ and 0 to 9.5 wt.% La₂O₃; and
b) the quantity of Me(II)O given is formed from 0 to 3.5 wt.% CaO, 0 to 4.5 wt.% BaO and 0 to 5.0 wt.% MgO.

2. Glass ceramic according to Claim 1, **characterised in that** the quantities of some components, independently of one another, are as follows:
| Component | wt.% |
|---|---|
| SiO₂ | 52.0 to 61.0 |
| Al₂O₃ | 8.5 to 11.0 |
| La₂O₃ | 0 to 2.0 |
| K₂O | 0 to 15.0 |
| Na₂O | 6.0 to 15.0 |
| Li₂O | 0 to 4.0 |
| ZnO 3.6 to 7.0, in particular | 4.0 to 7.0 |
| CaO | 0.5 to 3.5 |
| BaO | 1.0 to 4.5 |
| TiO₂ | 0 to 2.8 |
| ZrO₂ | 0.5 to 5.0 |
| P₂O₅ | 0 to 2.0 |

3. Glass ceramic according to Claim 1 or 2 **characterised in that** it is essentially free from B₂O₃.

4. Glass ceramic according to one of Claims 1 to 3 **characterised in that** it comprises leucite crystals.

5. Glass ceramic according to Claim 4 **characterised in that** the leucite crystals have an average size of less than 5µm, relative to the number of crystals.

6. Glass ceramic according to one of Claims 1 to 5 **characterised in that** it comprises as additives dyes, fluorescent agents, other glasses, ceramics, other glass ceramics, opacifiers and/or stabilisers.

7. Glass ceramic according to one of Claims 1 to 6 **characterised in that** it has a linear thermal expansion coefficient of 8.0 to 18.7 x 10⁻⁶K⁻¹, measured in the range from 100 to 400°C.

8. Process for the production of the glass ceramic according to one of Claims 1 to 7 **characterised in that** a glass is produced, which comprises the components according to Claim 1, and the produced glass is then subjected to a heat treatment at 600 to 900°C over a period of 30 minutes to 5 hours.

9. Process according to Claim 8 **characterised in that** prior to the heat treatment, the produced glass is ground and sieved to give a powder with a grain size of less than 90µm.

10. Dental material comprising alkali-zinc-silicate glass and glass ceramic according to Claim 1, wherein the alkali-zinc-silicate glass comprises the components according to Claim 1.

11. Use of the glass ceramic according to one of Claims 1 to 7 as dental material or constituent of dental material.

12. Use according to Claim 11, wherein the glass ceramic or the glass is used as correction material for metal ceramic or all-ceramic dental superstructures.

13. Use according to Claim 11 or 12, wherein the dental superstructure has the shape of a crown, a bridge or a partial crown.

14. Use according to one of Claims 11 to 13, wherein the glass ceramic is used as correction material for inlays.

15. Use according to one of Claims 11 to 13, wherein the glass ceramic is used as veneering material for all-ceramic or metallic dental superstructures or those present in the form of alloys.

16. Moulded dental product, which has a content of glass ceramic according to one of Claims 1 to 7.

## Revendications

1. Vitrocéramique alcali-zinc-silicate, **caractérisée en ce qu'**elle contient les composants ci-dessous :
| Composant | % en poids |
|---|---|
| SiO₂ | 52,0 à 63,5 |
| Me(III)₂O₃ | 8,5 à 13,0 |
| K₂O | 0 à 20,5 |
| Na₂O | 1,5 à 20,0 |
| Li₂O | 0 à 5,0 |
| ZnO 2,0 à 8,0, en particulier | 3,1 à 8,0 |
| Me(II)O | 2,5 à 6,5 |
| TiO₂ + ZrO₂ | 0,5 à 6,0 |
| SnO₂ | 0 à 9,5 |
| P₂O₅ | 0 à 4,0 |
| F | 0 à 2,0 |
| CeO₂ | 0 à 3,0 |
a) la quantité indiquée de Me(III)₂O₃ étant constituée de 0 à 13 % en poids d'Al₂O₃ et de 0 à 9,5 % en poids de La₂O₃ ; et
b) la quantité indiquée de Me(II)O étant constituée de 0 à 3,5 % en poids de CaO, de 0 à 4,5 % en poids de BaO, et de 0 à 5,0 % en poids de MgO.

2. Vitrocéramique selon la revendication 1, **caractérisée en ce que** les quantités de chacun des composants sont les suivantes, indépendamment les uns des autres :
| Composant | % en poids |
|---|---|
| SiO₂ | 52,0 à 61,0 |
| Al₂O₃ | 8,5 à 11,0 |
| La₂O₃ | 0 à 2,0 |
| K₂O | 0 à 15,0 |
| Na₂O | 6,0 à 15,0 |
| Li₂O | 0 à 4,0 |
| ZnO 3,6 à 7,0, en particulier | 4,0 à 7,0 |
| CaO | 0,5 à 3,5 |
| BaO | 1,0 à 4,5 |
| TiO₂ | 0 à 2,8 |
| ZrO₂ | 0,5 à 5,0 |
| P₂O₅ | 0 à 2,0 |

3. Vitrocéramique selon la revendication 1, **caractérisée en ce qu'**elle est sensiblement exempte de B₂O₃.

4. Vitrocéramique selon l'une des revendications 1 à 3, **caractérisée en ce qu'**elle présente des cristaux de leucite.

5. Vitrocéramique selon la revendication 4, **caractérisée en ce que** les cristaux de leucite ont une taille moyenne inférieure à 5 µm, rapportée au nombre de cristaux.

6. Vitrocéramique selon l'une des revendications 1 à 5, **caractérisée en ce qu'**elle contient comme additifs des colorants, des agents fluorescents, d'autres verres, des céramiques, d'autres vitrocéramiques, des opacifiants et/ou des stabilisants.

7. Vitrocéramique selon l'une des revendications 1 à 6, **caractérisée en ce qu'**elle présente un coefficient de dilatation thermique linéaire de 8,0 à 18,7 x 10⁻⁶ K⁻¹ mesuré dans une plage de 100 à 400° C.

8. Procédé de fabrication de la vitrocéramique selon l'une des revendications 1 à 7, **caractérisé en ce qu'**un verre contenant les composants selon la revendication 1 est fabriqué, et **en ce que** le verre fabriqué est ensuite soumis à un traitement thermique à une température comprise entre 600 et 900° C pendant une durée de 30 minutes à 5 heures.

9. Procédé selon la revendication 8, **caractérisé en ce que** le verre fabriqué est broyé et tamisé avant le traitement thermique pour obtenir une poudre d'une granulométrie inférieure à 90 µm.

10. Matériau dentaire contenant du verre alcali-zinc-silicate et de la vitrocéramique selon la revendication 1, le verre alcali-zinc-silicate contenant les composants selon la revendication 1.

11. Utilisation de la vitrocéramique selon l'une des revendications 1 à 7 comme matériau dentaire ou composant d'un matériau dentaire.

12. Utilisation selon la revendication 11, la vitrocéramique ou le verre étant utilisés comme matériau de correction pour des suprastructures dentaires céramo-métalliques ou tout céramique.

13. Utilisation selon l'une des revendications 11 ou 12, la suprastructure dentaire étant sous forme de couronne, de bridge ou de couronne partielle.

14. Utilisation selon l'une des revendications 11 à 13, la vitrocéramique étant utilisée comme matériau de correction pour des inlays.

15. Utilisation selon l'une des revendications 11 ou 13, la vitrocéramique étant utilisée comme matériau de parement pour des suprastructures dentaires tout céramique présentes sous forme, métallique, ou d'alliage.

16. Produit dentaire formé contenant de la vitrocéramique selon l'une des revendications 1 à 7.
